# EUROPEAN PATENT SPECIFICATION

(11) **EP 3 214 180 B1**
(45) Date of publication and mention of the grant of the patent: **17.11.2021**
(21) Application number: 16194450.9
(22) Date of filing: 18.10.2016
(51) Int. Cl.: C12Q 1/22, C12Q 1/30

(54) **METHODS, COMPOSITIONS AND KITS FOR DETERMINING CLEANNESS OF A SURFACE**
VERFAHREN, ZUSAMMENSETZUNGEN UND KITS ZUR BESTIMMUNG DER SAUBERKEIT EINER OBERFLÄCHE
PROCÉDÉS, COMPOSITIONS ET KITS PERMETTANT DE DÉTERMINER LA PROPRETÉ D'UNE SURFACE

(30) Priority: 01.03.2016 US 201662301716 P
(43) Date of publication of application: 06.09.2017
(73) Proprietor: Sani-Marc Inc., Victoriaville, Quebec G6P 7E3 (CA)
(72) Inventor: DESROSIERS, Dominic, Sainte-Eulalie, Québec G0Z 1E0 (CA); MARCHAND, Patrick, Victoriaville, Québec G6P 8B5 (CA)
(74) Representative: Icosa

(56) References cited:
- EP-A1- 0 105 747
- EP-A1- 2 902 497
- CN-A- 101 324 630
- US-A1- 2009 208 996
- US-B1- 6 479 454
- Netty Martowitono: "EFFICIENCY OF CLEANING AND DISINFECTION ON FISH CONTACT SURFACES TABLE OF CONTENTS", , 1 January 2011 (2011-01-01), pages 1-25, XP055355473, Retrieved from the Internet: URL:http://www.unuftp.is/static/fellows/do cument/netty2011prf.pdf [retrieved on 2017-03-16]
- Aat Bioquest: "Amplite(TM) Fluorimetric Catalase Assay Kit *Red Fluorescence* Ordering Information Storage Conditions Instrument Platform", , 1 June 2012 (2012-06-01), pages 1-3, XP055355486, Retrieved from the Internet: URL:http://www.interchim.fr/ft/G/GCX680.pd f [retrieved on 2017-03-16]
- M BARTOSZEK ET AL: "The Study of pH Influence on Bovine Liver Catalase by Means of UV-VIS Spectroscopy and Spin Labelling Method", POLISH J. OF ENVIRON. STUD. 4A, vol. 15, 1 January 2006 (2006-01-01), pages 41-43, XP055496996,

## Description

### RELATED APPLICATION

This application claims priority to United States provisional application No. 62/301,716 filed on March 1, 2016.

### FIELD OF THE INVENTION

The invention relates to the field of hygiene and more particularly to surface cleaning.

### BACKGROUND OF THE INVENTION

In many highly contaminated environments such as hospitals, restaurants, food processing facilities, slaughterhouses, etc., it is extremely important to make sure that cleaning has been done properly. For instance, nosocomial infections are a major problem in hospitals in Canada and around the world. Every year, in Canada alone, more than 200 000 patients will get a nosocomial infection and nearly ten thousand of these patients will die as a result. Surface cleanness is also critical in other environments such as laboratories where the contaminants may include not only bacteria and viruses, but also chemicals and radioactive compounds.

One of the reasons for the inadequate cleaning is the lack of suitable tools that would ensure better control of the quality of cleaning. Indeed, microorganisms and radioactivity are not visible to the naked eye and it is extremely difficult to assess cleanness. Because it is difficult to ensure that the cleaning has been done properly, some areas to be cleaned may be neglected, intentionally or not.

Some tools already exist to try to ensure adequate cleaning of a surface. For instance, there are different methods in which one will make some "spot check" in order to detect the presence of remaining bacteria or viruses on surfaces that have been previously cleaned. These may include taking smears for later analysis in a laboratory or marking the surface with a pen having an ink detectable under UV light. However, these methods have numerous deficiencies including the high number of smears that have to be taken to cover a large surface, the delays and cost associated with the samples than have to be analyzed in a remote place or with special equipment, the risks of cross-contaminations, etc.

The present invention concerns methods, compositions and kits for determining the cleanness of a surface that are based on the reaction of decomposition of hydrogen peroxide by catalase:

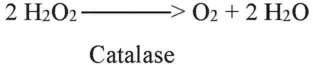

The above chemical reaction is well known and it has been applied by others for the detection of microorganism and biofilms, for instance US patent 7,764,602; International PCT publication WO 2014/019182; European patent publications EP 0 105 747 and EP 2 902 497, and French patent publication FR 2 611 744. However, the approaches described in these patent documents have different limitations, notably the fact that they rely on the presence of catalase in the bacteria. Accordingly, these are all dependent on the presence of catalase-positive bacteria and they are of no utility for detecting microorganisms that have undetectable level of catalase or that produce no catalase at all, let alone the detection of contaminated radioactive surfaces.

There is thus a need for improved approaches and techniques for determining the cleanness of a surface, and more particularly surfaces from contaminated environments like those found in hospitals, restaurants, food processing facilities, slaughterhouses, laboratories, etc.

There is also a need for reliable, effective and cheap approaches for improving surface hygiene and assessing cleanness of a surface that may have been contaminated by different types of microorganisms (e.g. bacteria, viruses, yeast, etc.) or by any other type of contaminants (e.g. radioactivity, chemicals, proteins, fat, blood, urine, excrement, biological matters, mineral deposits, etc.).

The present invention addresses these needs, as it relates to methods, compositions and kits for determining cleanness of a surface.

Features of the invention will be apparent from review of the disclosure, drawings and description of the invention below.

### BRIEF SUMMARY OF THE INVENTION

The invention relates to a method, compositions and a kit for determining the cleanness of a surface.

According toone aspect, the invention relates to a kit for determining the cleanness of a surface, the kit comprising an enzymatic solution comprising catalase, wherein the enzymatic solution comprises a pH between 4 and 11, and a developer solution comprising hydrogen peroxide wherein the enzymatic solution and/or the developer solution is (are) contained in a handheld vaporizer or handheld spray bottle.

According to another aspect, the invention relates to a method for determining the cleanness of a surface comprising:
- applying on a zone of a surface to be cleaned an enzymatic solution comprising catalase and letting it dry;
- after cleaning said surface, applying on said zone a developer solution comprising hydrogen peroxide; and
- detecting a catalytic reaction between remaining catalase and hydrogen peroxide, wherein presence of a catalytic reaction is indicative of a surface not properly cleaned.

According to one embodiment, the enzymatic solution and/or developer solution is(are) sprayed. The enzymatic solution is let dry for at least 15 sec., preferably at least 30 sec., preferably at least 1 min., preferably at least 90 sec., preferably at least 2 min., preferably at least 5 min., preferably at least 10 min., preferably at least 15 min., preferably at least 20 min., preferably at least 30 min., preferably at least 60 min. According to one embodiment, said catalytic reaction is detectable at the naked-eye, said catalytic reaction causing formation of bubbles and/or a foam. According to one embodiment, the method of invention is for assessing proper removal from an allegedly cleaned surface of at least one of microorganisms, chemicals, radioactive compounds, biological materials and minerals, and wherein said allegedly cleaned surface is found in an hospital, a restaurant, a food processing facility, a slaughterhouse or a laboratory.

The enzymatic solution comprises catalase from 40 U/g to 900 000 U/g, preferably from 400 U/g to 750 000 U/g, preferably from 1 000 U/g to 500 000 U/g, more preferably from 4 000 U/g to 20 000 U/g.

The enzymatic solution may also comprise one or more solvents. The enzymatic solution may also comprise one or more components including, but not limited to surfactants (ionic or non-ionic), buffers, preservatives, stabilizers and thickening agents. According to one embodiment, the enzymatic solution comprises one or more solvents selected from the group consisting of methanol, ethanol and isopropanol, and wherein the enzymatic solution comprises solvent from 5% w/w to 65% w/w; preferably from 25% w/w to 50% w/w. According to one embodiment, the enzymatic solution comprises an ionic surfactant and/or a non-ionic surfactant, and wherein the enzymatic solution comprises surfactant from 0.01% w/w to 5% w/w, preferably from 0.01% w/w to 2.5% w/w, more preferably from 0.05% w/v to 0.5% w/w. According to one embodiment, the enzymatic solution further comprises at least one of a buffer, a preservative, a stabilizer and a thickening agent; and wherein the developer solution further comprises at least one of a dye, a buffer, a preservative, a stabilizer and a thickening agent. According to one embodiment, the enzymatic solution comprises a pH between 4.5 and 7.5, preferably between 5.7 and 6.3, more preferably of about 6.0; and wherein the developer solution comprises a pH between 4 and 9, preferably between 6 and 8, more preferably of about 7.5.

The developer solution comprises hydrogen peroxide from 0.05 % w/w to 50% w/w, preferably from 0.5 % w/w to 10 % w/w, more preferably from 1% w/w to 5 % w/w.

The developer solution may also comprise one or more components including, but not limited to, foaming agents, dyes, buffers, preservatives, stabilizers and thickening agents. According to one embodiment, the developer solution comprises one or more foaming agents selected from the group consisting of decylamine oxide, lauramine oxide, myristyl dimethylamine oxide, and cocoamidopropylamine oxide, and wherein the developer solution comprises foaming agent from 0.05% w/w to 10 % w/w, preferably from 0.1% w/w to 5% w/w, more preferably between 0.5% w/w to 1.5 % w/w.

The invention also relates to particular enzymatic and developer solutions like the ones defined hereafter in **Table 1A, Table 1B, Table 2A, Table 2B, Table 3,** and **Table 4.** According to one embodiment, the enzymatic solution is selected from:
▪ Composition A consisting of:
   - from 0.001% to 19%w/w of catalase corresponding to about 42 U/g to about 806 000 U/g;
   - from 0% to 10% w/w of propylene glycol;
   - from 0% to 0.01% w/w of chloro-2-methyl-4-isothiazolin-3-one;
   - from 0.01% to 5% w/w of ethoxylated C12-C15 alcohols; and
   - from 0% to 99.999% w/w of solvent selected from isopropanol, demineralized water and mixture thereof;
▪ Composition B consisting of:
   - 0.11% w/w of catalase corresponding to 4780 U/g;
   - 1% w/w of propylene glycol;
   - 0.0004% w/w of chloro-2-methyl-4-isothiazolin-3-one;
   - 0.085% w/w of ethoxylated C12-C15 alcohols;
   - 30% w/w of isopropanol;
   - 68.9146% w/w of demineralized water; and
   - wherein pH is adjusted to about 6;
▪ Composition C consisting of:
   - 33.305 g of demineralized water;
   - 15 g of isopropanol;
   - 1 g of propylene glycol;
   - 0.015 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
   - 0.05 g of a fatty alcohol ethoxylate solution 85% w/w; and
   - 0.63 g of a catalase solution having an activity of 200 000 CIU/g;
▪ Composition D consisting of:
   - 48.305 g of demineralized water;
   - 1 g of propylene glycol;
   - 0.015 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
   - 0.05 g of a fatty alcohol ethoxylate solution 85% w/w; and
   - 0.63 g of a catalase solution having an activity of 200 000 CIU/g;
▪ Composition E consisting of:
   - 33.355 g of demineralized water;
   - 15 g of isopropanol;
   - 1 g of propylene glycol;
   - 0.015 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water; and
   - 0.63 g of a catalase solution having an activity of 200 000 CIU/g;
▪ Composition F consisting of:
   - 68.62 g of demineralized water;
   - 30 g of isopropanol;
   - 0.03 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
   - 0.1 g of a fatty alcohol ethoxylate solution 85% w/w; and
   - 1.25 g of a catalase solution having an activity of 200 000 CIU/g;
▪ Composition G consisting of:
   - 68.12 g of demineralized water;
   - 30 g of isopropanol;
   - 0.5 g of propylene glycol;
   - 0.03 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
   - 0.1 g of a fatty alcohol ethoxylate solution 85% w/w; and
   - 1.25 g of a catalase solution having an activity of 200 000 CIU/g;
▪ Composition H consisting of:
   - 67.62 g of demineralized water;
   - 30 g of isopropanol;
   - 1 g of propylene glycol;
   - 0.03 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
   - 0.1 g of a fatty alcohol ethoxylate solution 85% w/w; and
   - 1.25 g of a catalase solution having an activity of 200 000 CIU/g;
▪ Composition I consisting of:
   - 67.12 g of demineralized water;
   - 30 g of isopropanol;
   - 1.5 g of propylene glycol;
   - 0.03 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
   - 0.1 g of a fatty alcohol ethoxylate solution 85% w/w; and
   - 1.25 g of a catalase solution having an activity of 200 000 CIU/g;
▪ Composition J consisting of:
   - 66.62 g of demineralized water;
   - 30 g of isopropanol;
   - 2 g of propylene glycol;
   - 0.03 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
   - 0.1 g of a fatty alcohol ethoxylate solution 85% w/w; and
   - 1.25 g of a catalase solution having an activity of 200 000 CIU/g;
   and
▪ Composition K consisting of:
   - 66.12 g of demineralized water;
   - 30 g of isopropanol;
   - 2.5 g of propylene glycol;
   - 0.03 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
   - 0.1 g of a fatty alcohol ethoxylate solution 85% w/w; and
   - 1.25 g of a catalase solution having an activity of 200 000 CIU/g.

According to one embodiment, the developer solution is selected from:
▪ Composition L consisting of:
   - from 0% to 2% w/w of xanthan gum;
   - from 0% to 1% w/w of sodium hydroxide;
   - from 0.5% to 50% w/w of hydrogen peroxide;
   - from 0.001% to 5% w/w of 1-hydroxyethylidiene-1,1-diphosphonic acid (HEDP);
   - from 0.05% to 10% w/w of amine oxide; and
   - demineralized water as the solvent to complete to 100%;
   and
▪ Composition M consisting of:
   - 0.4% w/w of xanthan gum;
   - 0.4% w/w of sodium hydroxide;
   - 3% w/w of hydrogen peroxide;
   - 0.8% w/w of 1-hydroxyethylidiene-1,1-diphosphonic acid (HEDP);
   - 0.9% w/w of amine oxide; and
   - 94.5% w/w of demineralized water having a pH adjusted to about 7.5.

The kit, method and compositions according to the invention may assist in assessing proper removal of microorganisms (e.g. bacteria, viruses, yeast, etc.), chemicals, or radioactive compounds, biological materials (e.g. food, proteins, fat, blood, urine, excrements, and other biological matters), minerals(e.g. mineral deposits) and the like from an allegedly cleaned surface. The surface may be found for instance in hospitals, restaurants, food processing facilities, slaughterhouses and laboratories.

As it will be appreciated, the present invention possess numerous advantages, including, but not limited to: ease of use; cost effectiveness; allowing a "real-time" visual detection; does not damage the surfaces; easy cleaning; prevent cross-contamination; provides aqueous solutions that are stable at a broad range of temperature (e.g. about -15°C to about 40°C) and provides for a fast evaporation of the enzymatic solution.

### BRIEF DESCRIPTION OF THE DRAWINGS (OR FIGURES)

In order for the invention to be readily understood, embodiments of the invention are illustrated by way of example in the accompanying drawings.
**Figure 1** is a flow chart illustrating the steps of a method for determining cleanness of a surface, according to one embodiment of the present invention.
**Figure 2** is a panel showing pictures of a particular zone of a surface which has been sprayed by an enzymatic solution and by a developer solution, according to one embodiment the invention. The sprayed zone is delimited by a rectangle defined with a tape. **(A)** = surface freshly sprayed with the enzymatic solution; **(B)** = surface on which the enzymatic solution has dried; **(C)** = uncleaned surface freshly sprayed with the developer solution; **(D)** = cleaned surface freshly sprayed with the developer solution.
**Figure 3** is a line graph showing time of evaporation of the enzymatic solution, according to the formulations of **Table 2:** Formulation 2294.27 (■); Formulation 3294.27 (●); Formulation 1296.27 (▲).
**Figure 4** is a line graph showing catalase activity over time, in the enzymatic solution according to the formulations of **Table 3**, comprising Propylene glycol at 0% (-•-), 0.5% (-▲-), 1% (·■·), 1.5% (·-◆-·), 2% (-●··) and 2.5% (·▲·).

Further details of the invention and its advantages will be apparent from the detailed description included below.

### DETAILED DESCRIPTION OF PREFERRED EMBODIMENTS

In the following description of the embodiments, references to the accompanying drawings are by way of illustration of an example by which the invention may be practiced.

The invention pertains to methods, compositions and kits for determining the cleanness of a surface that are based on the decomposition of hydrogen peroxide by catalase.

Briefly, according to the principles of the present invention cleanness of a surface is assessed by "soiling" a zone of the surface to be cleaned with catalase and, after cleaning of the surface has been done, detecting presence of residual catalase on the allegedly cleaned surface.

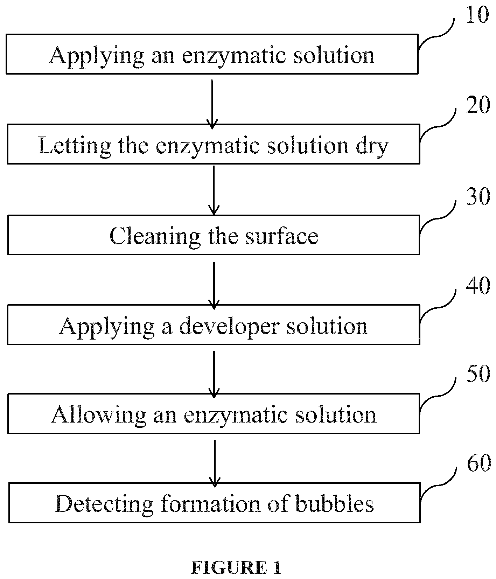

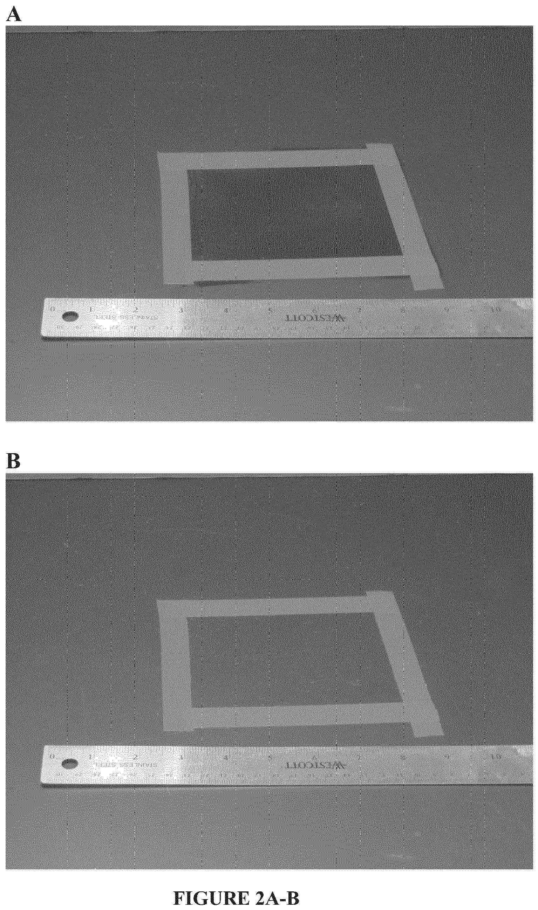

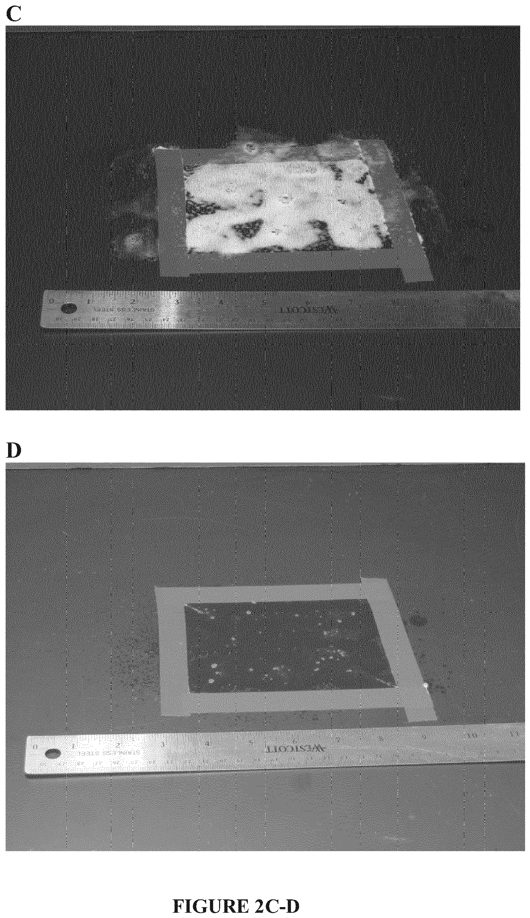

Referring to **Figure 1** and **Figure 2****,** prior to cleaning by a housekeeping person or concierge, a first person (e.g. supervisor or controller) applies an enzymatic solution comprising catalase on a given zone of the surface to be cleaned (10) (see also **Fig. 2A****).**

The applied enzymatic solution is let dried (20) so that it becomes practically invisible, as shown in **Fig. 2B**. The housekeeping person or concierge is let to do his job (30) and, after cleaning has been performed, the first person applies a developer solution (40) comprising hydrogen peroxide on the same zone of the allegedly cleaned surface. If the surface has not been cleaned properly, the hydrogen peroxide will react with remaining catalase (50), causing the formation of "bubbles" or a foam that will be easily visible to the naked eye (60) as shown in **Fig. 2C****.** To the contrary, if the surface is perfectly clean there will be no catalase left and no bubble will form as shown in **Fig. 2D****.**

### Enzymatic solution

One essential component of the enzymatic solution is a source of catalase. As is known, catalase is a common enzyme found in nearly all living organisms exposed to oxygen (such as bacteria, plants, and animals). It catalyzes the decomposition of hydrogen peroxide to water and oxygen as illustrated hereinbefore. According to the invention, the catalase may be obtained from different sources including, but not limited to, fungi (e.g. *Aspergillus niger, Aspergillus oryzae),* liver (e.g. bovine), plants and bacteria. The catalase may be obtained from commercial sources such as Sigma Aldrich, Novozymes (e.g. Catazyme™ 25L, Terminox™ Ultra 50L, Terminox™ Ultra 200L) and other suppliers like VWR, Fisher scientific, Dupont, etc. Terminox™ Ultra 200L is a catalase solution having an activity of 200 000 CIU/g.

The enzymatic activity of the catalase may be measured in enzyme unit (U). As used herein, one U is defined as the amount of catalase enzyme that catalyzes the conversion of 1 micro mole of hydrogen peroxide per minute in water, at pH 7, at 25°C, at a concentration of 26 mM hydrogen peroxide.

In embodiments, the enzymatic solution comprises about 40 U/g to about 900 000 U/g catalase, about 400 U/g to about 750 000 U/g catalase, or about 1 000 U/g to about 500 000 U/g catalase, or about 4 000 U/g to about 20 000 U/g catalase.

The enzymatic solution may also comprise one or more solvents to accelerate evaporation. Examples of envisioned solvents include, but are not limited to, methanol (MeOH), ethanol (EtOH) and isopropanol (isopropyl alcohol or IPA). In embodiments, the enzymatic solution comprises about 5% w/w to about 65% w/w solvent or about 25% w/w to about 50% w/w solvent.

The enzymatic solution may also comprise a surfactant (ionic or non-ionic) to accelerate evaporation. As is known, surfactants improve evaporation by reducing the tension of surface causing the product to extend more easily over the surface. Examples of envisioned ionic surfactants include, but are not limited to, sodium dodecyl sulfate (Stepanol™ WA Extra), sodium dodecylbenzene sulfonate (Bio-Soft™ S-101), sodium linear olefin sulfonate (Bio-Terge™ AS-40K), sodium caprylyl sulfonate (Bio-Terge™ PAS-8S), sodium laureth sulfate (Steol™ CS-230). Examples of envisioned non-ionic surfactants include, but are not limited to, linear ethoxylated alcohols (e.g. Bio-Soft™ N1-7, Bio-Soft™ 91-6, Bio-Soft™ E678). Bio-Soft™ E678 is a fatty alcohol ethoxylate solution 85% w/w. In embodiments, the enzymatic solution comprises between 0.01% w/w to 5% w/w surfactant, or between 0.01% w/w to 2.5% w/w surfactant, or between 0.05% w/v to 0.5% w/w surfactant.

The pH of the enzymatic solution may vary between 4 and 11. The pH may be selected in accordance with the pH optimum for the particular catalase in the solution and, preferably, also for avoiding damaging the surfaces. In embodiments, the pH is preferably between 4.5 and 7.5 or more preferably between 5.7 and 6.3, or even more preferably about 6.0.

To maintain a proper pH, the enzymatic solution may comprise a buffer. Examples of envisioned buffers include, but are not limited to, citrate buffer, phosphate buffer and phosphate citrate buffer. In embodiments, the enzymatic solution comprises about 0.05% w/w to about 5% w/w buffer, or about 0.1% w/w to about 2% w/w buffer, or about 0.5% w/w to about 1.5% w/w buffer.

The enzymatic solution may also comprise one or more preservatives. Examples of envisioned preservatives include, but are not limited to, chloro-2-methyl-4-isothiazolin-3-one (e.g. Kathon™ CG/ICP). Kathon™ CG/ICP II is an aqueous solution comprising 1.15% w/w 5-chloro-2-methyl-4-isothiazolin-3-one, 0.35% w/w2-methyl-4-isothiazolin-3-one, 2.80% w/w magnesium salts, 0.15% w/w cupric nitrate and 95.55% w/w water.

In embodiments, the enzymatic solution comprises about 0.01 ppm to about 100 ppm stabilizer, or about 0.05 ppm to about 50 ppm stabilizer, or about 0.1 ppm to about 10 ppm stabilizer.

The enzymatic solution may also comprise one or more stabilizers, including stabilizer(s) of catalase activity. Examples of envisioned stabilizers include, but are not limited to, propylene glycol, boric acid. In embodiments, the enzymatic solution comprises about 0.01% w/w to about 10% w/w stabilizer, or about 0.1% w/w to about 5% w/w stabilizer, or about 0.5% w/w to about 2% w/w stabilizer.

The enzymatic solution may comprise one or more thickening agents in order to improve adhesion of the enzymatic solution on vertical surfaces. Examples of envisioned thickening agents include, but are not limited to, Xanthan gum (e.g. Kelzan™ T), cross-linked polyacrylic acid polymer (e.g. Ultrez™ 10). In embodiments, the enzymatic solution comprises about 0.05% w/w to about 2% w/w thickening agent, or about 0.05% w/w to about 1% w/w thickening agent, or about 0.1% w/w to about 0.5% w/w thickening agent.

According to one particular embodiment, the enzymatic solution according to the invention is composed according to the following table:

**Table 1A: Enzymatic solution**

| **Components** | **CAS number** | **Role** | **Concentration (% w/w)** |
|---|---|---|---|
| Catalase | 9001-05-2 | enzyme | 0.001― 19 (about 42 U/g to about 806 000 U/g) |
| Propylene glycol | 57-55-6 | solvent /stabilizer | 0 - 10 |
| Chloro- 2-methyl-4-isothiazolin-3-one | 26172-55-4 | preservative | 0 ― 0.01 |
| Ethoxylated C12-15 alcohols | 68131-39-5 | surfactant | 0.01-5 |
| Isopropanol | 67-63-0 | solvent | 0 - 99.999 (To complete to 100% with water, if any) |
| Demineralized water | - | solvent | 0 ― 99.999 (To complete to 100% with isopropanol, in any) |

According to a preferred embodiment, the enzymatic solution according to the invention is composed according to the following table:

**Table 1B: Enzymatic solution**

| **Components** | **CAS number** | **Role** | **Concentration (% w/w)** |
|---|---|---|---|
| Catalase | 9001-05-2 | enzyme | 0.11 (4780 U/g) |
| Propylene glycol | 57-55-6 | solvent /stabilizer | 1 |
| Chloro- 2-methyl-4-isothiazolin-3-one | 26172-55-4 | preservative | 0.0004 |
| Ethoxylated C12-15 alcohols | 68131-39-5 | surfactant | 0.085 |
| Isopropanol | 67-63-0 | solvent | 30 |
| Demineralized water | - | solvent | 68.9146 |
| pH | Adjusted to about 6 | | |

The enzymatic solution may be formulated as an aqueous solution or as a powder for later dissolution in a suitable aqueous solution (e.g. water). The enzymatic solution may be formulated as a ready to use solution or as a liquid concentrate (e.g. 2X, 3X, 4X, 5X, 10X etc.) for further dilution. For a composition formulated as a liquid and/or solid ingredient may be mixed with a predetermined volume of filtered or distilled water. If necessary, the resulting mixed solution may be adjusted to the desired pH by addition of suitable acidifying agents.

### Developer solution

A related aspect of invention relates to a developer solution. As indicated hereinbefore, the role of the developer solution is to provide a source of hydrogen peroxide to react with catalase provided by the enzymatic solution.

Accordingly, one essential component of the developer solution is a source of hydrogen peroxide. In embodiments, the developer solution comprises about 0.05% w/w to about 50% w/w hydrogen peroxide, or about 0.5% w/w to about 10% w/w hydrogen peroxide, or about 1% w/w to about 5% w/w hydrogen peroxide.

The developer solution may also comprise one or more thickening agents in order to improve adhesion of the developer solution on vertical surfaces. Examples of envisioned thickening agents include, but are not limited to, Xanthan gum (e.g. Kelzan™ T), cross-linked polyacrylic acid polymer (e.g. Ultrez™ 10). In embodiments, the developer solution comprises between 0.05% w/w to 2% w/w thickening agent, or between 0.1% w/w to 1% w/w thickening agent, or between 0.2% w/w to 0.8% w/w thickening agent.

The pH of the developer solution may vary between 4 and 11. The pH may be selected in accordance with the pH optimum for the hydrogen peroxide stability and, preferably, also for avoiding damaging the surfaces. In embodiments, the pH is preferably between 4 and 9 and more preferably between 6 and 8, or even more preferably about 7.5.

The developer solution may also comprise one or more foaming agents in order to improve visual effect of developer solution on detection. Examples of envisioned foaming agent include, but are not limited to decylamine ocide (e.g. Ammonyx™ DO), lauramine oxide (e.g. Ammonyx™ LO), myristyl dimethylamine oxide (e.g. Ammonyx™ MO), cocoamidopropylamine oxide (e.g. Ammonyx™ CDO special). In embodiments, the developer solution comprises about 0.05% w/w to about 10% w/w foaming agent, or about 0.1% w/w to 5% w/w about foaming agent, or about 0.5% w/w to about 1.5 % w/w foaming agent.

The developer solution may also comprise one or more stabilizers in order to improve the stability of the developer solution. Examples of envisioned stabilizer include, but are not limited to 1-hydroxyethylidiene-1,1-diphosphonic acid (HEDP) (e.g. Dequest™ 2010). In embodiments, the developer solution comprises about 0.001% w/w to about 5% w/w stabilizer, or about 0.01% w/w to about 3% w/w stabilizer, or about 0.05% w/w to about 1% w/w stabilizer.

The developer solution may also comprise one or more dyes in order to improve the contrast (e.g. visual detection) between the foam and the product. Examples of envisioned dye include, but are not limited to disodium 6-hydroxy-5-[(2-methoxy-5-methyl-4-sulfophenyl)azo]-2-naphthalenesulfonate (FD&C red 40), ethyl - [4 - [[4 - [ethyl -[(3 - sulfophenyl) methyl] amino] phenyl] - (2 - sulfophenyl) methylidene] - 1 - cyclohexa - 2, 5 - dienylidene] - [(3 - sulfophenyl) methyl] azanium (FD&C blue 1), ethyl - [4 - [[4 - [ethyl -[(3 - sulfophenyl) methyl] amino] phenyl] - (4 - hydroxy - 2 - sulfophenyl) methylidene] - 1 - cyclohexa - 2, 5 - dienylidene] - [(3 - sulfophenyl) methyl] azanium (FD&C Green 3). In embodiments, the developer solution comprises about 5 ppm to about 100 ppm dye, or about 10 ppm to about 75 ppm dye, or about 20 ppm to about 50 ppm dye.

According to one particular embodiment, the developer solution according to the invention is composed according to the following table:

**Table 2A: Developer solution**

| **Components** | **CAS number** | **Role** | **Concentration (% w/w)** |
|---|---|---|---|
| Xanthan gum | 11138-66-2 | Ticking agent | 0 ― 2 |
| Sodium hydroxide | 1310-73-2 | base | 0 ― 1 |
| Hydrogen Peroxide | 7722-84-1 | oxidizer | 0.5 ― 50 |
| HEDP | 2809-21-4 | Stabilizer | 0.001― 5 |
| Amine oxide | 61788-90-7 | Foaming agent | 0.05 ― 10 |
| Demineralized water | - | solvent | to complete to 100% |

According to a preferred embodiment, the developer solution according to the invention is composed according to the following table:

**Table 2B: Developer solution**

| **Components** | **CAS number** | **Role** | **Concentration (% w/w)** |
|---|---|---|---|
| Xanthan gum | 11138-66-2 | Ticking agent | 0.4 |
| Sodium hydroxide | 1310-73-2 | base | 0.4 |
| Hydrogen Peroxide | 7722-84-1 | oxidizer | 3 |
| HEDP | 2809-21-4 | Stabilizer | 0.8 |
| Amine oxide | 61788-90-7 | Foaming agent | 0.9 |
| Demineralized water | - | solvent | 94.5 |
| pH | Adjusted to about 7.5 | | |

The developer solution may be formulated as an aqueous solution or as a powder for later dissolution in a suitable aqueous solution (e.g. water). The developer solution may be formulated as a ready to use solution or as a liquid concentrate (e.g. 2X, 3X, 4X, 5X, 10X etc.) for further dilution. For a composition formulated as a liquid and/or solid ingredient may be mixed with a predetermined volume of filtered or distilled water. If necessary, the resulting mixed solution may be adjusted to the desired pH by addition of suitable acidifying agents.

### Vaporization

The enzymatic solution and/or the developer solution are applied on the surface by vaporization or spray. The solution(s) is(are) contained in a handheld vaporizer or handheld spray bottle capable of delivering preferably a mist of fine liquid particles on the surface.

The vaporizer or spray bottle will distribute a sufficient amount to allow a quick and visually easy detection. In one embodiment, a handheld vaporizer or handheld spray bottle is used to distribute about 0.25 ml to about 0.30 ml of solution in one spray. Preferably the nozzle and quantity distributed is such that the spray will cover a diameter of at least about 0.5 cm to about 60 cm, preferably about 5 cm or 30 cm, when sprayed at a distance of about 2 to 40 cm of the surface.

Regarding the enzymatic solution, the amount to be applied on the surface should not be too high in order to avoid a slow evaporation of the solution. Indeed, the greater the amount of solution, the longer the time of evaporation to dryness. Also, if the amount of solution is too high the solution will tend to drip when applied to vertical surfaces. In embodiments, the enzymatic solution and developer solution may comprise one or more thickening agents in order to improve adhesion of the enzymatic solution on vertical surfaces. In embodiments, the enzymatic solution is let dry for at least 15 sec. or at least 30 sec., or at least 1 min., or at least 90 sec., or at least 2 min. or at least 5 min. or at least 10 min., or at least 15 min., or at least 20 min., or at least 30 min, or at least 60 min or more. It may also be envisioned to let dry the enzymatic solution for many hours or days, before cleaning the surface and/or applying the developer solution on the surface.

### Kit

A further aspect of the invention relates to kits, e.g. cleanness detection kits. The kit of the invention may be useful for the practice of the methods of the invention, particularly for determining cleanness of a surface in hospitals, restaurants, food processing facilities, slaughterhouses, laboratories, etc. as described herein.

The kit of the invention comprises the following components: (i) an enzymatic solution comprising catalase, and (ii) a developer solution comprising hydrogen peroxide, as described hereinbefore, wherein the enzymatic solution and/or the developer solution is (are) contained in a handheld vaporizer or a handheld spray bottle.

The kit may also comprise additional components, including but not limited to: a user manual or instructions, a spray bottle, pen(s), marking sheets, boxes, holders, cleaning solutions, wipes, etc. Theinvention is further illustrated by the following examples, which should not be construed as further or specifically limiting.

### EXAMPLES

The examples set forth herein below provide exemplary methods and results showing development, feasibility and utility of the methods, compositions and/or kits, according to the invention.

### Example 1: Accelerating evaporation of the enzymatic solution

A study was carried out to assess evaporation of the enzymatic solution in presence or absence of a solvent and/or a surfactant. The following formulations were prepared and tested:

**Table 3: Enzymatic solution**

| | **Formulation number** | | |
|---|---|---|---|
| | **2294.27** | **3294.27** | **1296.27** |
| Demineralized water | 33.305 g | 48.305 g | 33.355 g |
| Isopropanol | 15 g | - | 15 g |
| Propylene glycol | 1 g | 1 g | 1 g |
| Kathon™ CG/ICP II (1.25%) | 0.015 g | 0.015 g | 0.015 g |
| Bio-Soft™ E678 (85%) | 0.05 g | 0.05 g | 0 g |
| Terminox™ Ultra 200L | 0.63 g | 0.63 g | 0.63 g |

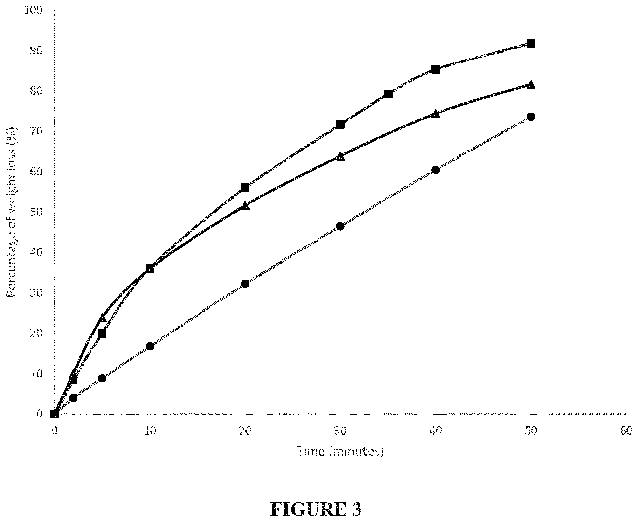

The results of the tests are illustrated in **Figure 3****.** As can be seen, the speed of evaporation increased in presence of a surfactant (Bio-Soft™ E678; formulation no. 2294.27 vs. no. 1296.27). Also, presence of a solvent (isopropanol) increased the evaporation (formulation no. 2294.27 vs. no. 3294.27).

### Example 2: Long term stability of the enzymatic solution

A study was carried out to assess long term stability of the enzymatic solution, in presence of various concentrations of a stabilizer. The following formulations were prepared and tested at room temperature:

**Table 4: Enzymatic solutions**

| | **Formulation number** | | | | | |
|---|---|---|---|---|---|---|
| | **1299.27** | **2299.27** | **3299.27** | **4299.27** | **5299.27** | **6299.27** |
| Demineralized water | 68.62 g | 68.12 g | 67.62 g | 67.12 g | 66.62 g | 66.12 g |
| Isopropanol | 30 g | 30 g | 30 g | 30 g | 30 g | 30 g |
| Propylene glycol | 0 g | 0.5 g | 1 g | 1.5 g | 2g | 2.5 g |
| Kathon™ CG/ICP II (1.25%) | 0.03 g | 0.03 g | 0.03 g | 0.03 g | 0.03 g | 0.03 g |
| Bio-Soft™ E678 (85%) | 0.1 g | 0.1 g | 0.1 g | 0.1 g | 0.1 g | 0.1 g |
| Terminox™ Ultra 200L | 1.25 g | 1.25 g | 1.25 g | 1.25 g | 1.25 g | 1.25 g |

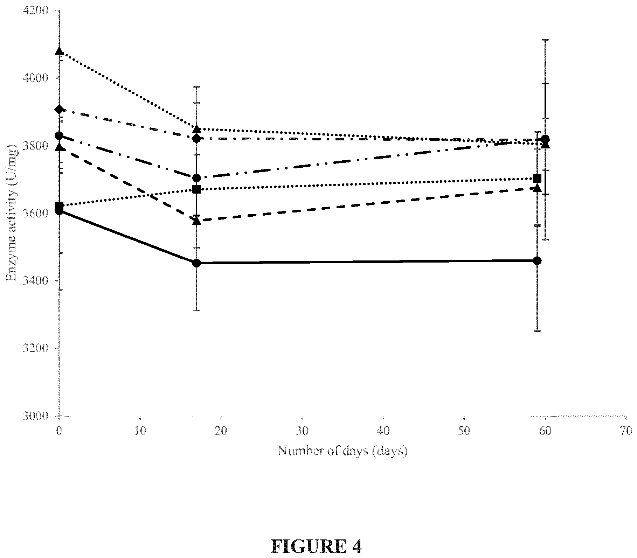

The results of the tests are illustrated in **Figure 4****.** As can be seen, enzymatic activity is relatively stable at all the tested concentrations of propylene glycol. It was calculated that the combined average loss of activity over 60 days was about 100 U, or only about 2.6%, a value within the experimental error of the measurements.

Using a diluted solution comprising 2.5 times less catalase (i.e. equivalent to a "loss" of 60% of the original activity), visual detection with the developer solution was still possible due to the formation of bubbles and a foam. However, the speed of the reaction was slower.

Altogether, these results confirm that the enzymatic solution is stable in the long term and useful for real-life and commercial applications (i.e. long term stability required through the steps of manufacture, shipping, storage, distanced repeated uses, etc.).

Headings are included herein for reference and to aid in locating certain sections. These headings are not intended to limit the scope of the concepts described therein, and these concepts may have applicability in other sections throughout the entire specification.

The singular forms "a", "an" and "the" include corresponding plural references unless the context clearly dictates otherwise. Thus, for example, reference to "a compound" includes one or more of such compounds, and reference to "the method" includes reference to equivalent steps and methods known to those of ordinary skill in the art that could be modified or substituted for the methods described herein.

Unless otherwise indicated, all numbers expressing quantities of ingredients, reaction conditions, concentrations, properties, and so forth used in the specification and claims are to be understood as being modified in all instances by the term "about". At the very least, each numerical parameter should at least be construed in light of the number of reported significant digits and by applying ordinary rounding techniques. Accordingly, unless indicated to the contrary, the numerical parameters set forth in the present specification and attached claims are approximations that may vary depending upon the properties sought to be obtained. Notwithstanding that the numerical ranges and parameters setting forth the broad scope of the embodiments are approximations, the numerical values set forth in the specific examples are reported as precisely as possible. Any numerical value, however, inherently contains certain errors resulting from variations in experiments, testing measurements, statistical analyses and such.

It is understood that the examples and embodiments described herein are for illustrative purposes only.

## Claims

1. A kit for determining the cleanness of a surface comprising:
- an enzymatic solution comprising catalase wherein the enzymatic solution comprises a pH between 4 and 11; and
- a developer solution comprising hydrogen peroxide;
wherein the enzymatic solution and/or the developer solution is(are) contained in a handheld vaporizer or a handheld spray bottle.

2. The kit according to claim 1, wherein the enzymatic solution comprises catalase from 40 U/g to 900 000 U/g, preferably from 400 U/g to 750 000 U/g, preferably from 1 000 U/g to 500 000 U/g, more preferably from 4 000 U/g to 20 000 U/g.

3. The kit according to claim 1 or 2, wherein the enzymatic solution comprises one or more solvents selected from the group consisting of methanol, ethanol and isopropanol, and wherein the enzymatic solution comprises solvent from 5% w/w to 65% w/w, preferably from 25% w/w to 50% w/w.

4. The kit according to any one of claims 1 to 3, wherein the enzymatic solution comprises an ionic surfactant and/or a non-ionic surfactant, and wherein the enzymatic solution comprises surfactant from 0.01% w/w to 5% w/w, preferably from 0.01% w/w to 2.5% w/w, more preferably from 0.05% w/w to 0.5% w/w.

5. The kit according to any one of claims **1** to **4,** wherein the enzymatic solution further comprises at least one of a buffer, a preservative, a stabilizer and a thickening agent; and wherein the developer solution further comprises at least one of a dye, a buffer, a preservative, a stabilizer and a thickening agent.

6. The kit according to any one of claims **1** to **5,** wherein the enzymatic solution comprises a pH between 4.5 and 7.5, preferably between 5.7 and 6.3, more preferably of about 6.0; and wherein the developer solution comprises a pH between 4 and 9, preferably between 6 and 8, more preferably of about 7.5.

7. The kit according to any one of claims **1** to **6,** wherein the developer solution comprises hydrogen peroxide from 0.05% w/w to 50% w/w, preferably from 0.5% w/w to 10% w/w, more preferably from 1% w/w to 5% w/w.

8. The kit according to any one of claims **1** to **7,** wherein the developer solution comprises one or more foaming agents selected from the group consisting of decylamine oxide, lauramine oxide, myristyl dimethylamine oxide, and cocoamidopropylamine oxide, and wherein the developer solution comprises foaming agent from 0.05% w/w to 10% w/w, preferably from 0.1% w/w to 5% w/w, more preferably between 0.5% w/w to 1.5% w/w.

9. A method for determining the cleanness of a surface comprising:
- applying on a zone of a surface to be cleaned an enzymatic solution comprising catalase and letting it dry;
- after cleaning said surface, applying on said zone a developer solution comprising hydrogen peroxide; and
- detecting a catalytic reaction between remaining catalase and hydrogen peroxide, wherein presence of a catalytic reaction is indicative of a surface not properly cleaned.

10. The method according to claim **9,** wherein said enzymatic solution and/or developer solution are sprayed.

11. The method according to claim **9** or claim **10,** wherein said enzymatic solution is let dry for at least 15 sec., preferably at least 30 sec., preferably at least 1 min., preferably at least 90 sec., preferably at least 2 min., preferably at least 5 min., preferably at least 10 min., preferably at least 15 min., preferably at least 20 min., preferably at least 30 min., preferably at least 60 min.

12. The method according to any one of claims **9** to **11,** wherein said catalytic reaction is detectable at the naked-eye, said catalytic reaction causing formation of bubbles and/or a foam.

13. The method according to any one of claims **9** to **12,** for assessing proper removal from an allegedly cleaned surface of at least one of microorganisms, chemicals, radioactive compounds, biological materials and minerals, and wherein said allegedly cleaned surface is found in an hospital, a restaurant, a food processing facility, a slaughterhouse or a laboratory.

14. An enzymatic solution selected from:
▪ Composition A consisting of:
- from 0.001% to 19% w/w of catalase corresponding to about 42 U/g to about 806 000 U/g;
- from 0% to 10% w/w of propylene glycol;
- from 0% to 0.01% w/w of chloro-2-methyl-4-isothiazolin-3-one;
- from 0.01% to 5% w/w of ethoxylated C12-C15 alcohols; and
- from 0% to 99.999% w/w of solvent selected from isopropanol, demineralized water and mixture thereof;
▪ Composition B consisting of:
- 0.11% w/w of catalase corresponding to 4780 U/g;
- 1% w/w of propylene glycol;
- 0.0004% w/w of chloro-2-methyl-4-isothiazolin-3-one;
- 0.085% w/w of ethoxylated C12-C15 alcohols;
- 30% w/w of isopropanol;
- 68.9146% w/w of demineralized water; and
- wherein pH is adjusted to about 6;
▪ Composition C consisting of:
- 33.305 g of demineralized water;
- 15 g of isopropanol;
- 1 g of propylene glycol;
- 0.015 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
- 0.05 g of a fatty alcohol ethoxylate solution 85% w/w; and
- 0.63 g of a catalase solution having an activity of 200 000 CIU/g;
▪ Composition D consisting of:
- 48.305 g of demineralized water;
- 1 g of propylene glycol;
- 0.015 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
- 0.05 g of a fatty alcohol ethoxylate solution 85% w/w; and
- 0.63 g of a catalase solution having an activity of 200 000 CIU/g;
▪ Composition E consisting of:
- 33.355 g of demineralized water;
- 15 g of isopropanol;
- 1 g of propylene glycol;
- 0.015 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water; and
- 0.63 g of a catalase solution having an activity of 200 000 CIU/g;
▪ Composition F consisting of:
- 68.62 g of demineralized water;
- 30 g of isopropanol;
- 0.03 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
- 0.1 g of a fatty alcohol ethoxylate solution 85% w/w; and
- 1.25 g of a catalase solution having an activity of 200 000 CIU/g;
▪ Composition G consisting of:
- 68.12 g of demineralized water;
- 30 g of isopropanol;
- 0.5 g of propylene glycol;
- 0.03 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
- 0.1 g of a fatty alcohol ethoxylate solution 85% w/w; and
- 1.25 g of a catalase solution having an activity of 200 000 CIU/g;
▪ Composition H consisting of:
- 67.62 g of demineralized water;
- 30 g of isopropanol;
- 1 g of propylene glycol;
- 0.03 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
- 0.1 g of a fatty alcohol ethoxylate solution 85% w/w; and
- 1.25 g of a catalase solution having an activity of 200 000 CIU/g;
▪ Composition I consisting of:
- 67.12 g of demineralized water;
- 30 g of isopropanol;
- 1.5 g of propylene glycol;
- 0.03 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
- 0.1 g of a fatty alcohol ethoxylate solution 85% w/w; and
- 1.25 g of a catalase solution having an activity of 200 000 CIU/g;
▪ Composition J consisting of:
- 66.62 g of demineralized water;
- 30 g of isopropanol;
- 2 g of propylene glycol;
- 0.03 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
- 0.1 g of a fatty alcohol ethoxylate solution 85% w/w; and
- 1.25 g of a catalase solution having an activity of 200 000 CIU/g; and
▪ Composition K consisting of:
- 66.12 g of demineralized water;
- 30 g of isopropanol;
- 2.5 g of propylene glycol;
- 0.03 g of an aqueous solution comprising 1.15% w/w 5-chloro-2-methyl-4-isothiazolin-3-one, 0.35% w/w 2-methyl-4-isothiazolin-3-one, 2.80% w/w magnesium salts, 0.15% w/w cupric nitrate and 95.55% w/w water;
- 0.1 g of a fatty alcohol ethoxylate solution 85% w/w; and
- 1.25 g of a catalase solution having an activity of 200 000 CIU/g;

15. A developer solution selected from:
▪ Composition L consisting of:
- from 0% to 2% w/w of xanthan gum;
- from 0% to 1% w/w of sodium hydroxide;
- from 0.5% to 50% w/w of hydrogen peroxide;
- from 0.001% to 5% w/w of 1-hydroxyethylidiene-1,1-diphosphonic acid (HEDP);
- from 0.05% to 10% w/w of amine oxide; and
- demineralized water as the solvent to complete to 100%; and
▪ Composition M consisting of:
- 0.4% w/w of xanthan gum;
- 0.4% w/w of sodium hydroxide;
- 3% w/w of hydrogen peroxide;
- 0.8% w/w of 1-hydroxyethylidiene-1,1-diphosphonic acid (HEDP);
- 0.9% w/w of amine oxide; and
- 94.5% w/w of demineralized water having a pH adjusted to about 7.5.

## Patentansprüche

1. Kit zur Bestimmung der Sauberkeit einer Oberfläche, umfassend:
- eine enzymatische Lösung, die Katalase umfasst, wobei die enzymatische Lösung einen pH-Wert zwischen 4 und 11 umfasst; und
- eine Entwicklerlösung, die Wasserstoffperoxid umfasst;
wobei die enzymatische Lösung und/oder die Entwicklerlösung in einem tragbaren Zerstäuber oder einer tragbaren Sprühflasche enthalten ist (sind).

2. Kit nach Anspruch 1, wobei die enzymatische Lösung Katalase von 40 U/g bis 900.000 U/g, vorzugsweise von 400 U/g bis 750.000 U/g, vorzugsweise von 1.000 U/g bis 500.000 U/g, bevorzugter von 4.000 U/g bis 20.000 U/g umfasst.

3. Kit nach Anspruch 1 oder 2, wobei die enzymatische Lösung ein oder mehrere Lösungsmittel umfasst, die aus der Gruppe ausgewählt sind, die aus Methanol, Ethanol und Isopropanol besteht, und wobei die enzymatische Lösung Lösungsmittel von 5 Gew.-% bis 65 Gew.-%, vorzugsweise von 25 Gew.-% bis 50 Gew.-% umfasst.

4. Kit nach einem der Ansprüche 1 bis 3, wobei die enzymatische Lösung ein ionisches Tensid und/oder ein nicht ionisches Tensid umfasst, und wobei die enzymatische Lösung Tensid von 0,01 Gew.-% bis 5 Gew.-%, vorzugsweise von 0,01 Gew.-% bis 2,5 Gew.-%, bevorzugter von 0,05 Gew.-% bis 0,5 Gew.-% umfasst.

5. Kit nach einem der Ansprüche 1 bis 4, wobei die enzymatische Lösung weiter mindestens eines von einem Puffer, einem Konservierungsmittel, einem Stabilisator und einem Verdickungsmittel umfasst; und wobei die Entwicklerlösung weiter mindestens eines von einem Farbstoff, einem Puffer, einem Konservierungsmittel, einem Stabilisator und einem Verdickungsmittel umfasst.

6. Kit nach einem der Ansprüche 1 bis 5, wobei die enzymatische Lösung einen pH-Wert zwischen 4,5 und 7,5, vorzugsweise zwischen 5,7 und 6,3, bevorzugter von etwa 6,0 umfasst; und wobei die Entwicklerlösung einen pH-Wert zwischen 4 und 9, vorzugsweise zwischen 6 und 8, bevorzugter von etwa 7,5 umfasst.

7. Kit nach einem der Ansprüche 1 bis 6, wobei die Entwicklerlösung Wasserstoffperoxid von 0,05 Gew.-% bis 50 Gew.-%, vorzugsweise von 0,5 Gew.-% bis 10 Gew.-%, bevorzugter von 1 Gew.-% bis 5 Gew.-% umfasst.

8. Kit nach einem der Ansprüche 1 bis 7, wobei die Entwicklerlösung ein oder mehrere Schäumungsmittel umfasst, die aus der Gruppe ausgewählt werden, die aus Decylaminoxid, Lauraminoxid, Myristyldimethylaminoxid, und Kokoamidpropylaminoxid besteht, und wobei die Entwicklerlösung Schäumungsmittel von 0,05 Gew.-% bis 10 Gew.-%, vorzugsweise von 0,1 Gew.-% bis 5 Gew.-%, bevorzugter von 0,5 Gew.-% bis 1,5 Gew.-% umfasst.

9. Verfahren zum Bestimmen der Sauberkeit einer Oberfläche, umfassend:
- Anwenden auf einer Zone einer zu reinigenden Oberfläche einer Katalase umfassenden enzymatischen Lösung und trocknen lassen;
- Nach dem Reinigen der Oberfläche, Anwenden auf die Zone einer Entwicklerlösung, die Wasserstoffperoxid umfasst; und
- Erkennen einer katalytischen Reaktion zwischen verbleibender Katalase und Wasserstoffperoxid, wobei das Vorhandensein einer katalytischen Reaktion auf eine nicht sauber gereinigte Oberfläche hinweist.

10. Verfahren nach Anspruch 9, wobei die enzymatische Lösung und/oder Entwicklerlösung gesprüht wird (werden).

11. Verfahren nach Anspruch 9 oder Anspruch 10, wobei die enzymatische Lösung mindestens 15 Sek. lang, vorzugsweise 30 Sek. lang, vorzugsweise mindestens 1 Min. lang, vorzugsweise mindestens 90 Sek. lang, vorzugsweise mindestens 2 Min. lang, vorzugsweise mindestens 5 Min. lang, vorzugsweise mindestens 10 Min. lang, vorzugsweise mindestens 15 Min. lang, vorzugsweise mindestens 20 Min. lang, vorzugsweise mindestens 30 Min. lang, vorzugsweise mindestens 60 Min. lang trocknen lassen wird.

12. Verfahren nach einem der Ansprüche 9 bis 11, wobei die katalytische Reaktion mit freiem Auge erkennbar ist, wobei die katalytische Reaktion das Bilden von Bläschen und/oder von Schaum bewirkt.

13. Verfahren nach einem der Ansprüche 9 bis 12, zum Beurteilen von sauberem Entfernen von einer vermeintlich gereinigten Oberfläche von mindestens einem von Mikroorganismen, Chemikalien, radioaktiven Verbindungen, biologischen Stoffen und Mineralien, und wobei die vermeintlich gereinigte Oberfläche in einem Spital, einem Restaurant, einer lebensmittelverarbeitenden Fabrik, einem Schlachthof oder einem Labor vorgefunden wird.

14. Enzymatische Lösung, ausgewählt aus:
• Zusammensetzung A, bestehend aus:
- von 0,001 Gew.-% bis 19 Gew.-% an Katalase, entsprechend etwa 42 U/g bis etwa 806.000 U/g;
- von 0 Gew.-% bis 10 Gew.-% an Propylenglykol;
- von 0 Gew.-% bis 0,01 Gew.-% an Chlor-2-Methyl-4-Isothiazolin-3-on;
- von 0,01 Gew.-% bis 5 Gew.-% an ethoxylierten C12-C15 Alkoholen; und
- von 0 Gew.-% bis 99,999 Gew.-% an Lösungsmittel, ausgewählt aus Isopropanol, entmineralisiertem Wasser und einem Gemisch davon;
• Zusammensetzung B, bestehend aus:
- 0,11 Gew.-% an Katalase, entsprechend etwa 4.780 U/g;
- 1 Gew.-% an Propylenglykol;
- 0,0004 Gew.-% an Chlor-2-Methyl-4-Isothiazolin-3-on;
- 0,085 Gew.-% an ethoxylierten C12-C15 Alkoholen;
- 30 Gew.-% an Isopropanol;
- 68,9146 Gew.-% an entmineralisiertem Wasser; und
- wobei der pH-Wert auf etwa 6 angepasst wird;
• Zusammensetzung C, bestehend aus:
- 33,305 g an entmineralisiertem Wasser; und
- 15 g an Isopropanol;
- 1 g an Propylenglykol;
- 0,015 g an wässriger Lösung, umfassend 1,15 Gew.-% 5-Chlor-2-Methyl-4-Isothiazolin-3-on, 0,35 Gew.-% an 2-Methyl-4-Isothiazolin-3-on, 2,80 Gew.-% an Magnesiumsalzen, 0,15 Gew.-% an Kupfernitrat und 95,55 Gew.-% an Wasser;
- 0,05 g an Fettalkoholethoxylatlösung, 85 Gew.-%; und
- 0,63 g einer Katalaselösung, die eine Aktivität von 200.000 CIU/g aufweist;
• Zusammensetzung D, bestehend aus:
- 48,305 g an entmineralisiertem Wasser;
- 1 g an Propylenglykol;
- 0,015 g an wässriger Lösung, umfassend 1,15 Gew.-% 5-Chlor-2-Methyl-4-Isothiazolin-3-on, 0,35 Gew.-% an 2-Methyl-4-Isothiazolin-3-on, 2,80 Gew.-% an Magnesiumsalzen, 0,15 Gew.-% an Kupfernitrat und 95,55 Gew.-% an Wasser;
- 0,05 g an Fettalkoholethoxylatlösung, 85 Gew.-%; und
- 0,63 g einer Katalaselösung, die eine Aktivität von 200.000 CIU/g aufweist;
• Zusammensetzung E, bestehend aus:
- 33,355 g an entmineralisiertem Wasser;
- 15 g an Isopropanol;
- 1 g an Propylenglykol;
- 0,015 g an wässriger Lösung, umfassend 1,15 Gew.-% 5-Chlor-2-Methyl-4-Isothiazolin-3-on, 0,35 Gew.-% an 2-Methyl-4-Isothiazolin-3-on, 2,80 Gew.-% an Magnesiumsalzen, 0,15 Gew.-% an Kupfernitrat und 95,55 Gew.-% an Wasser;
- 0,63 g einer Katalaselösung, die eine Aktivität von 200.000 CIU/g aufweist;
• Zusammensetzung F, bestehend aus:
- 68,62 g an entmineralisiertem Wasser;
- 30 g an Isopropanol;
- 0,03 g an wässriger Lösung, umfassend 1,15 Gew.-% 5-Chlor-2-Methyl-4-Isothiazolin-3-on, 0,35 Gew.-% an 2-Methyl-4-Isothiazolin-3-on, 2,80 Gew.-% an Magnesiumsalzen, 0,15 Gew.-% an Kupfernitrat und 95,55 Gew.-% an Wasser;
- 0,1 g an Fettalkoholethoxylatlösung, 85 Gew.-%; und
- 1,25 g einer Katalaselösung, die eine Aktivität von 200.000 CIU/g aufweist;
• Zusammensetzung G, bestehend aus:
- 68,12 g an entmineralisiertem Wasser;
- 30 g an Isopropanol;
- 0,5 g an Propylenglykol;
- 0,03 g an wässriger Lösung, umfassend 1,15 Gew.-% 5-Chlor-2-Methyl-4-Isothiazolin-3-on, 0,35 Gew.-% an 2-Methyl-4-Isothiazolin-3-on, 2,80 Gew.-% an Magnesiumsalzen, 0,15 Gew.-% an Kupfernitrat und 95,55 Gew.-% an Wasser;
- 0,1 g an Fettalkoholethoxylatlösung, 85 Gew.-%; und
- 1,25 g einer Katalaselösung, die eine Aktivität von 200.000 CIU/g aufweist;
• Zusammensetzung H, bestehend aus:
- 67,62 g an entmineralisiertem Wasser;
- 30 g an Isopropanol;
- 1 g an Propylenglykol;
- 0,03 g an wässriger Lösung, umfassend 1,15 Gew.-% 5-Chlor-2-Methyl-4-Isothiazolin-3-on, 0,35 Gew.-% an 2-Methyl-4-Isothiazolin-3-on, 2,80 Gew.-% an Magnesiumsalzen, 0,15 Gew.-% an Kupfernitrat und 95,55 Gew.-% an Wasser;
- 0,1 g an Fettalkoholethoxylatlösung, 85 Gew.-%; und
- 1,25 g einer Katalaselösung, die eine Aktivität von 200.000 CIU/g aufweist;
• Zusammensetzung I, bestehend aus:
- 67,12 g an entmineralisiertem Wasser;
- 30 g an Isopropanol;
- 1,5 g an Propylenglykol;
- 0,03 g an wässriger Lösung, umfassend 1,15 Gew.-% 5-Chlor-2-Methyl-4-Isothiazolin-3-on, 0,35 Gew.-% an 2-Methyl-4-Isothiazolin-3-on, 2,80 Gew.-% an Magnesiumsalzen, 0,15 Gew.-% an Kupfernitrat und 95,55 Gew.-% an Wasser;
- 0,1 g an Fettalkoholethoxylatlösung, 85 Gew.-%; und
- 1,25 g einer Katalaselösung, die eine Aktivität von 200.000 CIU/ g aufweist;
• Zusammensetzung J, bestehend aus:
- 66,62 g an entmineralisiertem Wasser;
- 30 g an Isopropanol;
- 2 g an Propylenglykol;
- 0,03 g an wässriger Lösung, umfassend 1,15 Gew.-% 5-Chlor-2-Methyl-4-Isothiazolin-3-on, 0,35 Gew.-% an 2-Methyl-4-Isothiazolin-3-on, 2,80 Gew.-% an Magnesiumsalzen, 0,15 Gew.-% an Kupfernitrat und 95,55 Gew.-% an Wasser;
- 0,1 g an Fettalkoholethoxylatlösung, 85 Gew.-%; und
- 1,25 g einer Katalaselösung, die eine Aktivität von 200.000 CIU/g aufweist;
• Zusammensetzung K, bestehend aus:
- 66,12 g an entmineralisiertem Wasser;
- 30 g an Isopropanol;
- 2,5 g an Propylenglykol;
- 0,03 g an wässriger Lösung, umfassend 1,15 Gew.-% 5-Chlor-2-Methyl-4-Isothiazolin-3-on, 0,35 Gew.-% an 2-Methyl-4-Isothiazolin-3-on, 2,80 Gew.-% an Magnesiumsalzen, 0,15 Gew.-% an Kupfernitrat und 95,55 Gew.-% an Wasser;
- 0,1 g an Fettalkoholethoxylatlösung, 85 Gew.-%; und
- 1,25 g einer Katalaselösung, die eine Aktivität von 200.000 CIU/g aufweist;

15. Entwicklerlösung, ausgewählt aus:
• Zusammensetzung L, bestehend aus:
- von 0 Gew.-% bis 2 Gew.-% an Xanthangummi;
- von 0 Gew.-% bis 1 Gew.-% an Natriumhydroxid;
- von 0,5 Gew.-% bis 50 Gew.-% an Wasserstoffperoxid;
- von 0,001 Gew.-% bis 5 Gew.-% an 1-Hydroxyethylidien-1,1-Diphosphonsäure (HEDP);
- von 0,05 Gew.-% bis 10 Gew.-% an Aminoxid; und
- entmineralisiertem Wasser als Lösungsmittel zur Vervollständigung auf 100 %; und
• Zusammensetzung M, bestehend aus:
- 0,4 Gew.-% an Xanthangummi;
- 0,4 Gew.-% an Natriumhydroxid;
- 3 Gew.-% an Wasserstoffperoxid;
- 0,8 Gew.-% an 1-Hydroxyethylidien-1,1-Diphosphonsäure (HEDP);
- 0,9 Gew.-%an Aminoxid; und
- 94,5 Gew.-% an entmineralisiertem Wasser, das einen pH-Wert aufweist, der auf etwa 7,5 angepasst wird.

## Revendications

1. Une trousse pour déterminer la propreté d'une surface comprenant:
- une solution enzymatique comprenant de la catalase dans laquelle la solution enzymatique comprend un pH entre 4 et 11; et
- une solution révélatrice comprenant du peroxyde d'hydrogène;
dans laquelle solution enzymatique et/ou la solution révélatrice est(sont) contenue(s) dans un vaporisateur portatif ou un flacon pulvérisateur portatif.

2. La trousse selon la revendication 1, dans laquelle la solution enzymatique comprenant de la catalase de 40 U/g à 900 000 U/g, préférablement de 400 U/g à 750 000 U/g, préférablement de 1 000 U/g à 500 000 U/g, plus préférablement de 4 000 U/g à 20 000 U/g.

3. La trousse selon la revendication 1 ou 2, dans laquelle la solution enzymatique comprend un ou plusieurs solvants choisis dans le groupe constitué du méthanol, de l'éthanol et de l'isopropanol, et dans laquelle la solution enzymatique comprend un solvant de 5 % p/p à 65 % p/p, de préférence de 25 % p/p à 50 % p/p.

4. La trousse selon l'une quelconque des revendications 1 à 3, dans laquelle la solution enzymatique comprend un tensioactif ionique et/ou un tensioactif non ionique, et dans laquelle la solution enzymatique comprend un tensioactif de 0.01 % p/p à 5 % p/p, de préférence de 0.01 % p/p à 2.5 % p/p, plus préférablement de 0.05 % p/p à 0.5 % p/p.

5. La trousse selon l'une quelconque des revendications 1 à 4, dans laquelle la solution enzymatique comprend en outre au moins l'un d'un tampon, d'un conservateur, d'un stabilisant et d'un agent épaississant; et dans laquelle la solution révélatrice comprend en outre au moins l'un d'un colorant, d'un tampon, d'un agent de conservation, d'un stabilisant et d'un agent épaississant.

6. La trousse selon l'une quelconque des revendications 1 à 5, dans laquelle la solution enzymatique comprend un pH entre 4.5 et 7.5, de préférence entre 5.7 et 6.3, plus préférentiellement d'environ 6.0; et dans laquelle la solution révélatrice comprend un pH compris entre 4 et 9, de préférence entre 6 et 8, plus préférablement d'environ 7.5.

7. La trousse selon l'une quelconque des revendications 1 à 6, dans laquelle la solution révélatrice comprend du peroxyde d'hydrogène de 0.05 % p/p à 50 % p/p, de préférence de 0.5 % p/p à 10 % en p/p, plus préférablement de 1 % p/p à 5 % en p/p.

8. La trousse selon l'une quelconque des revendications 1 à 7, dans laquelle la solution révélatrice comprend un ou plusieurs agents moussants choisis dans le groupe constitué par l'oxyde de décylamine, l'oxyde de lauramine, l'oxyde de myristyl diméthylamine et l'oxyde de cocoamidopropylamine, et dans laquelle la solution révélatrice comprend un agent moussant de 0.05 % p/p à 10 % p/p, de préférence de 0.1 % p/p à 5 % p/p, plus préférablement entre 0.5 % p/p à 1.5 % p/p.

9. Procédé pour déterminer la propreté d'une surface comprenant :
- appliquer sur une zone d'une surface à nettoyer une solution enzymatique comprenant de la catalase et laisser sécher;
- après nettoyage de ladite surface, appliquer sur ladite zone une solution révélatrice comprenant du peroxyde d'hydrogène; et
- détecter une réaction catalytique entre la catalase restante et le peroxyde d'hydrogène, dans lequel la présence d'une réaction catalytique indique une surface mal nettoyée.

10. Le procédé selon la revendication 9, dans lequel ladite solution enzymatique et/ou solution révélatrice sont pulvérisées.

11. Procédé selon la revendication 9 ou la revendication 10. dans lequel ladite solution enzymatique est laissée à sécher pendant au moins 15 s, de préférence au moins 30 s, de préférence au moins 1 min., de préférence au moins 90 s, de préférence au moins 2 min, de préférence au moins 5 min, de préférence au moins 10 min, de préférence au moins 15 min, de préférence au moins 20 min, de préférence au moins 30 min, de préférence au moins 60 min.

12. Procédé selon l'une quelconque des revendications 9 à 11, dans lequel ladite réaction catalytique est détectable à l'œil nu, ladite réaction catalytique provoquant la formation de bulles et/ou d'une mousse.

13. Procédé selon l'une quelconque des revendications 9 à 12, pour évaluer l'élimination appropriée d'une surface prétendument nettoyée d'au moins l'un de micro-organismes, de produits chimiques, de composés radioactifs, de matières biologiques et de minéraux, et dans lequel ladite surface prétendument nettoyée se trouve dans un hôpital, un restaurant, une usine de transformation alimentaire, un abattoir ou un laboratoire.

14. Une solution enzymatique choisie parmi:
• Composition A constituée de:
- de 0.001 % à 19 % p/p de catalase correspondant à environ 42 U/g à environ 806 000 U/g;
- de 0 % à 10 % p/p de propylène glycol;
- de 0 % à 0.01 % p/p de chloro-2-méthyl-4-isothiazolin-3-one;
- de 0.01 % à 5 % p/p d'alcools éthoxylés en C12-C15; et
- de 0 % à 99.999 % p/p de solvant choisi parmi l'isopropanol, l'eau déminéralisée et leur mélange;
• Composition B constituée de:
- 0.11 % p/p de catalase correspondant à 4780 U/g;
- 1 % p/p de propylène glycol;
- 0.0004 % p/p de chloro-2-méthyl-4-isothiazoline-3-one;
- 0.085% p/p d'alcools éthoxylés en C12-C15;
- 30 % p/p d'isopropanol;
- 68.9146 % p/p d'eau déminéralisée; et
- dans laquelle le pH est ajusté à environ 6;
• Composition C constituée de:
- 33.305 g d'eau déminéralisée;
- 15 g d'isopropanol;
- 1 g de propylène glycol ;
- 0.015 g d'une solution aqueuse comprenant 1.15 % p/p de 5-chloro-2-méthyl-4-isothiazolin-3-one, 0.35 % p/p de 2-méthyl-4-isothiazolin-3-one, 2.80 % p/p sels de magnésium, 0.15 % p/p de nitrate cuivrique et 95.55 % p/p d'eau;
- 0.05 g d'une solution d'éthoxylate d'alcool gras à 85 % p/p; et
- 0.63 g d'une solution de catalase ayant une activité de 200 000 ClU/g;
• Composition D constituée de:
- 48,305 g d'eau déminéralisée;
- 1 g de propylène glycol;
- 0.015 g d'une solution aqueuse comprenant 1.15 % p/p de 5-chloro-2-méthyl-4-isothiazolin-3-one, 0.35 % p/p de 2-méthyl-4-isothiazolin-3-one, 2.80 % p/p sels de magnésium, 0.15 % p/p de nitrate cuivrique et 95.55 % p/p d'eau;
- 0.05 g d'une solution d'éthoxylate d'alcool gras à 85 % p/p; et
- 0.63 g d'une solution de catalase ayant une activité de 200 000 CIU/g;
• Composition E constituée de:
- 33.355 g d'eau déminéralisée;
- 15 g d'isopropanol;
- 1 g de propylène glycol;
- 0.015 g d'une solution aqueuse comprenant 1.15 % p/p de 5-chloro-2-méthyl-4-isothiazolin-3-one, 0.35 % p/p de 2-méthyl-4-isothiazolin-3-one, 2.80 % p/p de sels de magnésium, 0.15 % p/p de nitrate cuivrique et 95.55 % p/p d'eau; et
- 0.63 g d'une solution de catalase ayant une activité de 200 000 CIU/g;
• Composition F constituée de:
- 68,62 g d'eau déminéralisée;
- 30 g d'isopropanol;
- 0.03 g d'une solution aqueuse comprenant 1.15 % p/p de 5-chloro-2-méthyl-4-isothiazolin-3-one, 0.35% p/p de 2-méthyl-4-isothiazolin-3-one, 2.80 % p/p de sels de magnésium, 0.15 % p/p de nitrate cuivrique et 95.55 % p/p d'eau;
- 0.1 g d'une solution d'éthoxylate d'alcool gras à 85 % p/p; et
- 1.25 g d'une solution de catalase ayant une activité de 200 000 CIU/g;
• Composition G constituée de:
- 68,12 g d'eau déminéralisée;
- 30 g d'isopropanol;
- 0.5 g de propylène glycol;
- 0.03 g d'une solution aqueuse comprenant 1.15 % p/p de 5-chloro-2-méthyl-4-isothiazolin-3-one, 0.35% p/p de 2-méthyl-4-isothiazolin-3-one, 2.80 % p/p de sels de magnésium, 0.15 % p/p de nitrate cuivrique et 95.55 % p/p d'eau;
- 0.1 g d'une solution d'éthoxylate d'alcool gras à 85 % p/p; et
- 1.25 g d'une solution de catalase ayant une activité de 200 000 CIU/g;
• Composition H constituée de:
- 67,62 g d'eau déminéralisée;
- 30 g d'isopropanol;
- 1 g de propylène glycol;
- 0.03 g d'une solution aqueuse comprenant 1.15 % p/p de 5-chloro-2-méthyl-4-isothiazolin-3-one, 0.35% p/p de 2-méthyl-4-isothiazolin-3-one, 2.80 % p/p de sels de magnésium, 0.15 % p/p de nitrate cuivrique et 95.55 % p/p d'eau;
- 0.1 g d'une solution d'éthoxylate d'alcool gras à 85 % p/p; et
- 1.25 g d'une solution de catalase ayant une activité de 200 000 CIU/g;
• Composition I constituée de:
- 67,12 g d'eau déminéralisée;
- 30 g d'isopropanol;
- 1.5 g de propylène glycol;
- 0.03 g d'une solution aqueuse comprenant 1.15 % p/p de 5-chloro-2-méthyl-4-isothiazolin-3-one, 0.35% p/p de 2-méthyl-4-isothiazolin-3-one, 2.80 % p/p de sels de magnésium, 0.15 % p/p de nitrate cuivrique et 95.55 % p/p d'eau;
- 0.1 g d'une solution d'éthoxylate d'alcool gras à 85 % p/p; et
- 1.25 g d'une solution de catalase ayant une activité de 200 000 CIU/g;
• Composition J constituée de:
- 66,62 g d'eau déminéralisée;
- 30 g d'isopropanol;
- 2 g de propylène glycol;
- 0.03 g d'une solution aqueuse comprenant 1.15 % p/p de 5-chloro-2-méthyl-4-isothiazolin-3-one, 0.35% p/p de 2-méthyl-4-isothiazolin-3-one, 2.80 % p/p de sels de magnésium, 0.15 % p/p de nitrate cuivrique et 95.55 % p/p d'eau;
- 0.1 g d'une solution d'éthoxylate d'alcool gras à 85 % p/p; et
- 1.25 g d'une solution de catalase ayant une activité de 200 000 CIU/g; et
• Composition K constituée de:
- 66,12 g d'eau déminéralisée;
- 30 g d'isopropanol;
- 2.5 g de propylène glycol;
- 0.03 g d'une solution aqueuse comprenant 1.15 % p/p de 5-chloro-2-méthyl-4-isothiazolin-3-one, 0.35% p/p de 2-méthyl-4-isothiazolin-3-one, 2.80 % p/p de sels de magnésium, 0.15 % p/p de nitrate cuivrique et 95.55 % p/p d'eau;
- 0.1 g d'une solution d'éthoxylate d'alcool gras à 85 % p/p; et
- 1.25 g d'une solution de catalase ayant une activité de 200 000 CIU/g.

15. Une solution révélatrice sélectionnée parmi:
• Composition L constituée de:
- de 0 % à 2 % p/p de gomme xanthane;
- de 0 % à 1 % p/p d'hydroxyde de sodium;
- de 0.5 % à 50 % p/p de peroxyde d'hydrogène;
- de 0.001 % à 5 % p/p d'acide 1-hydroxyéthylidiène-1,1-diphosphonique (HEDP);
- de 0.05 % à 10 % p/p d'oxyde d'aminé; et
- de l'eau déminéralisée comme solvant pour compléter à 100 % ; et
• Composition M constituée de:
- 0.4 % p/p de gomme xanthane;
- 0.4 % p/p d'hydroxyde de sodium;
- 3 % p/p de peroxyde d'hydrogène;
- 0.8 % p/p d'acide 1-hydroxyéthylidiène-1,1-diphosphonique (HEDP);
- 0.9 % p/p d'oxyde d'aminé; et
- 94.5% p/p d'eau déminéralisée ayant un pH ajusté à environ 7.5.
